# EUROPEAN PATENT SPECIFICATION

(11) **EP 1 342 481 B1**
(45) Date of publication and mention of the grant of the patent: **21.10.2009**
(21) Application number: 03251316.0
(22) Date of filing: 05.03.2003
(51) Int. Cl.: A61M 5/142, A61M 5/168, A61M 5/172, A61M 27/00, A61M 31/00

(54) **Implantable drug delivery device**
Implantierbare Vorrichtung zur Arzneimittelverabreichung
Dispositif implantable pour l'administration de médicaments

(30) Priority: 06.03.2002 US 92954
(43) Date of publication of application: 10.09.2003
(73) Proprietor: Codman & Shurtleff, Inc., Raynham, Massachusetts 02767-0350 (US)
(72) Inventor: Rohr, William L., Marshfield, MA 02050 (US); Dextradeur, Alan L., Franklin, MA 02038 (US); Konieczynski, David D., Needham, MA 02492 (US)
(74) Representative: Mercer, Christopher Paul

(56) References cited:
- WO-A-01/35928
- DE-A- 10 108 251
- US-A- 5 011 472
- US-A- 5 643 207
- US-A- 5 720 720
- US-A- 6 134 474

## Description

### FIELD OF THE INVENTION

This invention pertains to an implantable device for delivery of a drug or bioactive material into a target tissue of a subject, such as to a locus in the subject's brain, or other desired organ or tissue location within a subject's body.

### BACKGROUND OF THE INVENTION

The treatment of a disease often involves prescribing one or more drugs for an afflicted individual. Medication schedules can vary depending upon a number of factors. There are conditions which require that different drugs be taken throughout the day. There are diseases which require that a patient take a series of medications throughout their lifetime. Some medical conditions require the direct administration of the drug to its target site, for example, because appropriate concentrations cannot be attained by systemic administration, or, as in the case of the central nervous system ("CNS"), systemic administration does not reach the target site. In all of these situations, it is important that drug delivery be accomplished in a rapid and efficient manner.

In the case of the CNS, it is not unusual that in many instances the direct route of administration is preferred. Ehrlich in 1885 described an almost impenetrable barrier to the CNS. This barrier is commonly referred to as the blood-brain barrier. This blood-brain barrier is selective with respect to what molecules can enter the CNS from the general circulation. As a result, many drugs used to treat CNS diseases cannot reach their intended target without modifying their structure in some manner.

To circumvent this difficulty of entry, direct administration of the drug into the CNS is often performed. One modality for accomplishing this is to use a syringe containing the desired drug and inject it into the cerebral spinal fluid ("CSF"), or into brain parenchyma. Alternatively, an implanted drug delivery system can be employed which uses an infusion catheter that is inserted within the CNS to directly deliver a material to the CSF or brain parenchyma. Often, the relevant drug or bioactive material is to be delivered to a localized region at extremely small dosage, and may be delivered at predetermined intervals throughout the day, or delivered in response to a physiological signal, e.g., in response to detection of a certain level of a protein, or a metabolite in the blood.

In many such systems a pump is activated to move the drug or bioactive material, usually contained in a physiological buffer, from a drug reservoir into an implanted infusion catheter, and the drug travels through the catheter until it is delivered to the target site. Once at the site, the drug is released from the catheter and enters the target tissue, typically by a diffusion mechanism. This delivery mechanism, typically involving an implanted delivery catheter, is especially useful for targeting tumors, wherein a chemotherapeutic or other treatment agent is to be selectively applied to the tumor. Other areas of active interest involve treatment of certain chronic or degenerative brain tissue conditions, and other conditions of the CNS. Several implantable infusion pumps have been proposed or developed for delivering a drug or bioactive material to the brain to effect various treatments.

The ability to implant a drug delivery system capable of selectively delivering multiple small doses of a drug, or doses of different drugs, has been realized in part also by the advent of microchip technology. A microchip device may include a plurality of drug reservoirs that are etched into or otherwise formed in a biocompatible implantable substrate, and are filled with the intended drug(s). A number of reservoirs are formed in a single microchip, and release of material from each reservoir is separately controlled, for example, by a barrier membrane or other controllable member that controllably effects release of the drug from the reservoir. This technology significantly enhances the versatility of implantable drug delivery technology. Reservoirs may be filled with different drugs, and the reservoirs can be capped with materials that either degrade or allow the drugs to diffuse passively out of the reservoir over time. Moreover, this capping material can be structured such that upon application of an electric potential, it erodes quickly, changes permeability or otherwise responds to the signal to release the active agent. The sites and times of this active release can then be controlled by a remote controller, by an integrally implanted programmed microprocessor, by an implanted but externally programmable unit, or other effective arrangement. The resulting power source and timing control can be compactly and robustly configured, resulting in an effective structure without the complex mechanical structure or large space requirements of a typical infusion pump.

Microchip drug devices are well suited to holding and dispensing multiple small doses of a drug or drugs. However, a limitation of current microchip drug delivery systems is that generally only small amounts of material enter the targeted organ, or permeate the targeted site within the organ. Typically, the drug is released from a reservoir housed within the microchip, and the drug travels into or over a target tissue region by a diffusion process, often competing against a clearance reaction having a rate which may be comparable to the release rate. This mode of delivery may therefore have an effective tissue penetration range of only a millimeter or less over many hours, or may result in substantially diminishing concentration as diffusion proceeds. The diffusion transport depends primarily on a free concentration gradient and the diffusivity of the dispensed drug in the target tissue. Generally, high molecular weight molecules such as antibodies tend to have a low diffusion rate, while low molecular weight molecules, although typically having greater diffusivity, are also susceptible to being cleared more quickly from the target site because of the ease of their entry into the capillary system. Thus for many potentially desirable applications, the penetrable/treatable tissue range and the treatment concentration profiles achievable by microchip delivery are severely limited.

WO 01/35928 A teaches apparatus and methods for the delivery of molecules to a site via carrier fluid according to the preamble of claim 1. The apparatus include microchip devices which have reservoirs containing the molecules for release. Embodiments also include systems for intravenous administration or drugs, wherein drug molecules are released from the microchip devices into a carrier fluid *ex vivo*, such as a saline solution, forming a drug/saline solution mixture, which is then delivered to a patient intravenously.

US-A-5 643 207 teaches an implantable system for infusing an agent into an organ containing an endogenous fluid, including an implantable reservoir for the agent and first and second catheters implanted into the organ. An implantable pump transmits the endogenous fluid to the organ through one catheter and returns it through the other catheter. A pre-determined quantity of the agent is added from the reservoir to the endogenous fluid to facilitate buttering and dilution of the agent before administration to the organ.

There remains a need for an efficient implantable drug delivery system effective to selectively deliver one or more drugs to a target site.

There also exists a need for a drug delivery system that extends the range and/or concentration profile for delivery of doses of drugs to the CNS.

### SUMMARY OF THE INVENTION

The invention is defined in claim 1. It comprises an implantable drug delivery system comprising a controlled release drug assembly, said drug assembly being configured for controllably releasing drug material.

The system differs from the prior art known from WO 01/35928 in that it includes a controller configured to initiate drug release out of phase with the operation of the infusion pump.

The controlled release drug assembly, positioned in or in communication with the fluid delivery line, releases a drug into the carrier fluid which is delivered by the infusion pump assembly, preferably at a rate effective to establish a local pressure gradient in the region of the discharge portion at the tissue site, so that the drug is delivered into, and preferably convectively driven by bulk transport, into the tissue at the target site. The carrier may be, e.g., a biologically inert or inactive fluid such as physiologic saline, or it may be an endogenous body fluid. Thus, the preferred infusion pump assembly advantageously provides a high flow infusion flow such that when the fluid bearing the drug exits the fluid delivery line near the target site, the drug undergoes convection driven transport and enhanced penetration into the target tissue site.

The discharge portion may be implanted providing a fixed site of drug administration, and may extend such that one or more ports open in the immediate vicinity of the target site, which may, for example, be a tumor site, a nerve, a lesion or other targeted region of affected brain or other tissue. The controlled release drug assembly and optionally the infusion pump may also be implanted, but these units need not be in the immediate vicinity of the target tissue. Thus, for example, when the target tissue is a brain lesion or tumor, the discharge portion may be stereotactically implanted in or adjacent the tumor through a cranial hole to release the carrier-borne drug in parenchymal spaces, while the other components of the system may be implanted subdermally, connected to the discharge portion by the fluid delivery line.

Thus, the controlled release drug assembly may be fitted in-line with the more proximal portion of the fluid delivery line such that the carrier fluid flow from the pump entrains material released from the release apparatus and the fluid then passes out via the port(s) of the fluid delivery line, or via an extension delivery conduit, at the target tissue site. The discharge portion assembly may be a needle-like assembly, such as a stainless steel or stiff polymer tube having elongated ports that release the drug over an extended site, while the fluid delivery line may be formed of one or more segments of polymer tubing of a suitable stiffness to dependably transmit microdose or microflow volumes of carrier from the pump through, past or over, the drug release device without loss of pressure. The pump may connect to several such fluid delivery lines, and these may have their distal ends implanted close to each other to enlarge the treated tissue volume of a single tissue region or organ; alternatively, the plural fluid delivery lines may be implanted at distinct sites. One or more sensors may be associated with the fluid delivery line or lines to report drug concentration, tissue condition or the like to a processor which may operate the pump and/or control the drug release assembly.

In some embodiments, the controlled release drug assembly may be formed in a wall of the fluid delivery line itself. For example, it may be implemented as a plurality of recessed sites constituting drug release reservoirs formed in the fluid release line wall, each site holding one or more desired drug(s) in a degradable polymer or other matrix material, or holding the drug separated from the fluid pathway by a degradable or a controlled porosity membrane, or in other controlled-release form. Alternatively, the controlled release drug assembly may be a separate unit, i.e., a microchip having a structure connected to but independent of the catheter. Another embodiment provides the drug release assembly as a replaceable release cartridge that fits within the fluid delivery line (e.g., a delivery catheter), and may be conveniently replaced when depleted without disturbing the implanted line. In some embodiments, the release assembly may couple to the fluid delivery line via a manifold or a set of separate passages effective to channel the fluid pumped by the infusion pump over or through all, or appropriate ones of, its release reservoirs and into the fluid delivery line.

The fluid supply to the inlet of the infusion pump may be an implanted reservoir or other supply. In one embodiment, a reservoir is implanted subdermally and possesses a cover or septum formed of a self-sealing polymer. The reservoir is refillable through the patient's skin by piercing the septum with a syringe to deliver a refill volume of the carrier fluid. The reservoir may also be a pressurized assembly, such as a pressure-driven bellows, in which case the infusion pump assembly may be implemented by a simply providing one or more valves, restrictors or other elements that regulate the time and/or the rate at which fluid is allowed to pass from the reservoir. Alternatively, the infusion pump may be an electrically powered assembly, having a power source and a controller. In accordance with another aspect of the invention, the pump may receive fluid from a fluid supply line or inlet catheter that is positioned to draw the body's endogenous fluid, for example, the patient's cerebrospinal fluid, into the pump as the carrier medium for drug delivery. This arrangement advantageously utilizes naturally compatible fluid, and requires neither a reservoir nor the periodic replenishment of the carrier. Moreover, when applied to an isolated body system such as the central nervous system, this embodiment advantageously at least partially offsets the volumes of fluid withdrawn and returned to the central nervous system, thus promoting isobaric fluid conditions in the skull or spinal column.

Other implantable drug delivery systems, which are not claimed, may include a chamber in the pump assembly that contains a concentrated delivery agent, which it supplies into the pumped carrier fluid. A mixing chamber may be provided to allow mixing of drugs before they are pumped to the tissue site. This is especially advantageous for multidrug regimens in which several incompatible or mutually unstable drugs are to be delivered at once, or in which concentration must be closely controlled.

### BRIEF DESCRIPTION OF THE DRAWINGS

These and other features of the invention will be understood from the description and claims below, taken together with the figures showing illustrative embodiments, wherein:
Figure 1 illustrates a first embodiment of a drug delivery system of the present invention;
Figure 1A illustrates a another embodiment of a drug delivery system of the present invention;
Figure 1B illustrates a another embodiment of a drug delivery system of the present invention;
Figure 2 illustrates another embodiment of a drug delivery system of the present invention;
Figure 2A illustrates another embodiment of a drug delivery system of the present invention;
Figure 3 illustrates another embodiment of a drug delivery system of the present invention; and
Figure 3A schematically illustrates another embodiment of a drug delivery system of the invention.

### DETAILED DESCRIPTION OF THE INVENTION

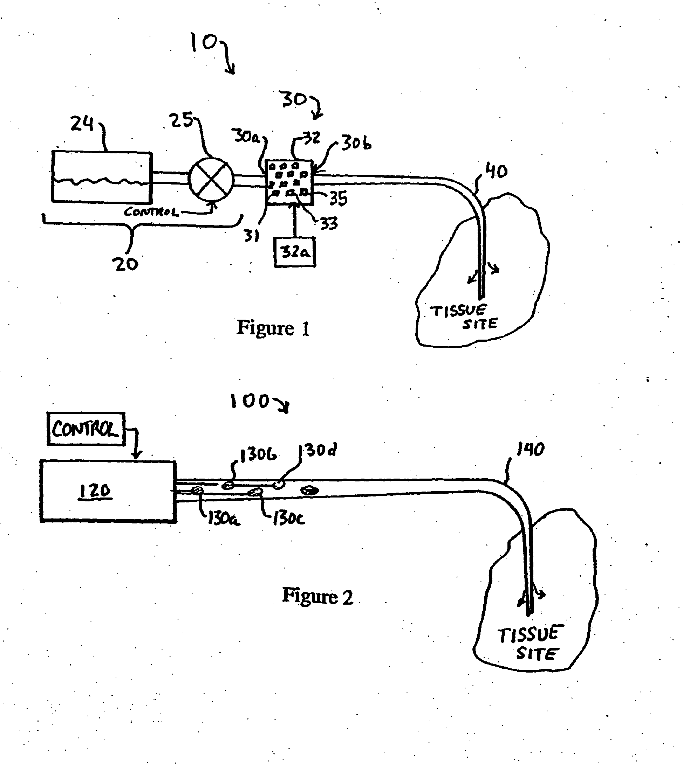

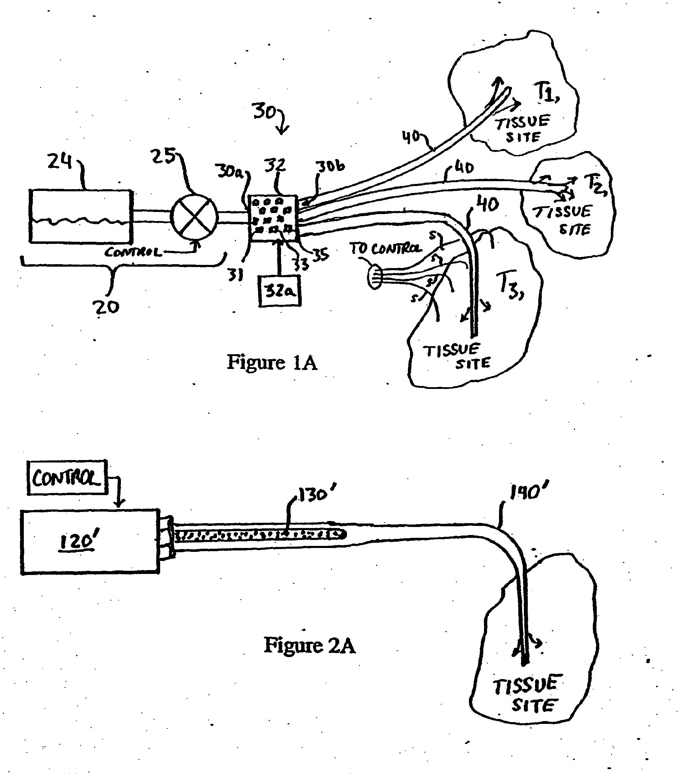

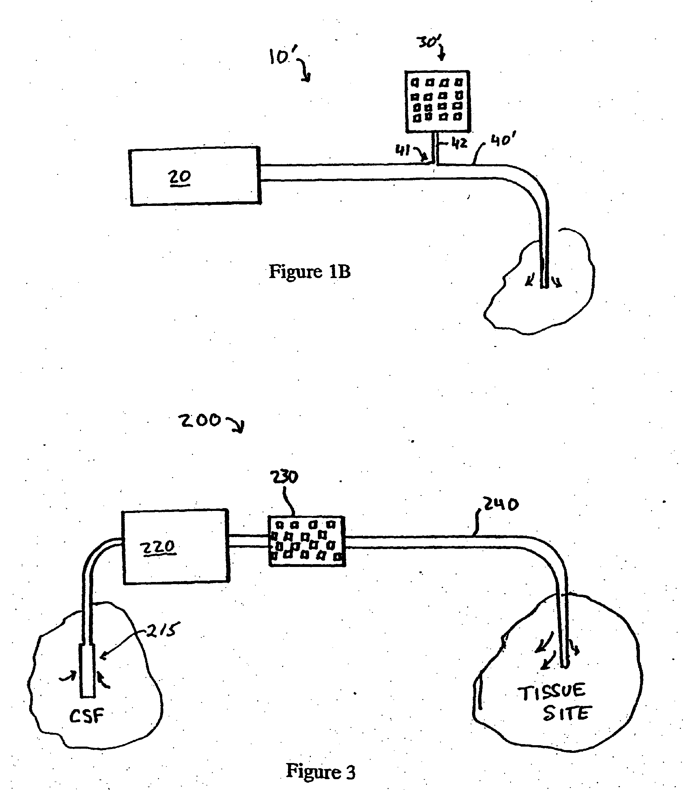

Figure 1 schematically illustrates a system 10 of the present invention for delivering a drug or treatment material to the central nervous system. The system includes an infusion reservoir or source 24 of fluid connected through a pump, valve or flow initiator/controller 25 to a controlled release drug delivery device (such as a microchip drug delivery device) 30 and then through a site delivery catheter 40 to deliver a drug, via ports at the distal end of the catheter, to a target tissue site. The system is configured to treat a localized tissue site in a patient's body, e.g., the central nervous system, and the catheter is implanted such that the fluid exits through one or more openings in its distal end region at the target tissue site and permeates through targeted tissue to effectively treat the targeted region. Thus, the region of effective tissue treatment is defined by the catheter placement. This may be placed at a site such as a brain tumor or diseased region of the brain, at a tumor to deliver a chemotherapeutic agent, at an organ to deliver an organ-specific treatment, at a nerve location to treat chronic pain, or at another suitable local site. The distal end portion of the catheter 40 may be a thin gauge needle-like tube having one or more elongated ports extending therealong. When the target tissue site is in the brain, catheter 40 may be implanted using a stereotactic frame to position it such that its ports lie adjacent to or extend centrally through the target site.

The reservoir 24 or supply and the pump, valve or controller 25 together form the infusion pump 20. The precise pump structure may be flexibly implemented with any suitable structure as known in the art, either with an electromechanically-actuated peristaltic or displacement pumping mechanisms, or with a pressurized reservoir or osmotically-driven source connected to a control valve or restrictor assembly to regulate the provision of fluid into the fluid delivery path. In either case, whether powered by pressure or electromechanically, the infusion pump assembly produces an accurately administered and sustainable flow of a total volume of fluid at a suitable infusion flow rate, discussed further below, such as a rate of about 0.5 to about 20.0 microliters per minute for a typical infusion delivery tube. The fluid itself may simply be a carrier, or it may include a drug or other active material within a carrier fluid. The carrier may be an inert or non-bioactive fluid, such as physiological buffer or saline, or may contain materials, such as adjuvants or the like to enhance use as a carrier and drug delivery vehicle. In one embodiment described below, the carrier may be a physiological fluid such as cerebrospinal fluid (CSF). One or more drugs or other bioactive materials are then provided directly into the flow path by the release unit 30.

The controlled release unit 30 delivers one or more drugs or other agents at controlled times or over controlled intervals. This unit may be a microchip device, for example, of the type shown in United States Patent 5,797,898 of Santini, Jr. *et al.* That is, unit 30 may be a miniaturized multi-well drug delivery device 32 with a plurality of reservoirs 31, 33, 35, and controller 32a configured to effect release from appropriate ones of the wells at appropriate times. The controller may operate in accordance with a programmed instruction set (via a fixed PROM) to operate at predetermined times, or may have a signal receiver to operate in response to instructions transmitted from outside the release assembly (e.g., via reception of signals transmitted from outside the patient's body). The release unit 30 may also, in some embodiments, respond to an input from a biosensor implanted within the patient's body to influence control over the drug release regimen.

As shown, one drug release unit 30 of the present invention has an inlet 30a that receives fluid from the pump, and an outlet 30b leading to the distal catheter. These adapt the controlled release unit so that when it releases its drugs, these enter the fluid pathway.

One suitable arrangement to adapt a microchip drug delivery device for such operation is to add a cover plate (not shown) over the unit 30, forming a flow manifold that channels fluid from the inlet 30a so that it passes over all or appropriate ones of the release sites 31, 33, 35, and to the outlet 30b. The manifold may be an active manifold, with different flow channels actively switched open or closed (for example, by micromechanical valve, electric field control or other means) to effectively channel fluid only over the desired release sites (when a passive release mechanism is used), or only over the device's current active release site(s) (when an electrically-actuated release structure is used). Alternatively, the manifold may be a passive assembly of sufficiently small volume and open shape that the carrier fluid provided at the inlet effectively washes over all reservoirs at once, receiving drugs from the activated release sites before passing to the outlet.

In accordance with one embodiment of the present invention, the pump assembly provides a flow of carrier fluid through the release device that is effective to increase the pressure locally at the region of the catheter distal outlet ports, where the catheter is implanted in tissue, creating a pressure gradient that drives bulk transport of the drug into the target tissue site. The carrier fluid thus serves as a quantifiable bulk medium for pressure delivery to move the small quantity of released drug to the tissue site and to establish a pressure gradient to enhance delivery at the tissue site. As such, the drug is delivered as a convection-enhanced, or pressure gradient-driven permeation of the target tissue, as described, for example, in United States Patent 5,720,720. For a typical implanted brain catheter delivery route, such pressure gradient transport may be achieved with a flow rate of about one-half (0.5) to about twenty (20.0) microliters per minute, preferably about 2.0 to 15.0 microliters/minute, and most preferably about ten microliters/minute (per implanted delivery tube). When the target site is another type of tissue, such as pancreatic tissue, the density and other features of the tissue will determine suitable delivery rates. These may, for example, vary when the target tissue is tumorous, in dependence upon the tumor tissue characteristics. It is expected that after suitable observations, the relevant properties may be correlated with or characterized by known data, such as the tumor type or stage.

Delivery of the drug or bioactive material may be sustained in cycles of several minutes, or may in some instances be continuous, or may be triggered for relatively short periods in response to detection of a condition. It will be understood that the precise rates and durations may depend upon a variety of factors, including the identity and concentration of the drug or bioactive material and carrier, the size and tissue properties of the target site, and the size of the delivery catheter and its ports, and the number of delivery catheters. Thus, flow rates above and below the indicated range may also be effective.

The pump flow may be set and the pump assembly actuated based upon modeled properties such as histological tissue traits, drug and fluid viscosity, catheter and port dimensions and the like, or the pump flow may be governed by one or more extrinsic inputs, e.g., by a controller operative on input signals from sensors that detect pressure or flow at relevant locations, or biosensors that provide other indicia relevant to selecting the rate for achieving and maintaining the desired drug delivery conditions.

An embodiment of the system may be formed using a conventional infusion pump for the pump assembly 20 as shown in Figure 1, and a controlled release drug delivery chip 32, with suitable flow segments for interconnecting the two units and for delivering the flow to the target tissue site. There may be more than one delivery catheter, and these may be connected to different target tissue sites T1, T2, T3 as shown in Figure 1A. The sites T1, T2, T3 may be selected on different sides (e.g., distributed around the perimeter) of a single targeted tissue site (e.g., a tumor, gland or organ) so as to maximize the effective treatment volume. Alternatively, the sites may be distinct, independently targeted sites, for example, to treat plural distinct lesions, tumors or disease sites in the brain. Systems of the invention may employ one or more sensors that provide output signals upon which the controller operates to determine a pumping or drug release regimen. The sensors may sense fluid pressure, detect the level or presence of a substance, a drug or a metabolite, or detect a physiologic condition to which the treatment is applied. Advantageously, an array of sensors may themselves be implanted at positions to determine the spatial distribution in the target tissue of the drug delivered by the delivery system, and a processor or controller may operate accordingly to achieve the desired dose or concentration distribution, or to achieve the desired control of sensed conditions during changing metabolic and tissue states. One such array of sensors is schematically shown in Figure 1A, and these may connect to the pump controller (if one is provided) and/or the controller of the release device (if present). Depending on the overall system configuration and the type of sensing elements, the sensor outputs may be subjected to various processing or simple thresholding operations, for detection of response conditions to which the system control is directed.

Advantageously, the invention may also be a system 100 as shown in Figure 2. In this system, an infusion pump 120 connects to a delivery catheter 140 that forms a flow path extending to and implanted in parenchymal tissue. A plurality of controlled release drug reservoirs 130a, 130b are formed directly in the wall of the catheter. The reservoirs may be actuated by control electrodes connected with control signal leads (not shown) embedded in the catheter wall. This construction has the advantage that dead space and lag time are minimized, and the flow path may have a short length. Thus, the flow conditions are well suited to assure that the released drugs are reliably entrained in the pumped flow of carrier fluid without the need for a specially-designed manifold to interface the flow with the geometry of the drug release unit, and without requiring a special chip geometry to enhance mixing. Drug release may be initiated out of phase with pumping, to assure that the drug is released into the fluid residing in the catheter before the flow is initiated, so that a higher concentration is achieved in the carrier fluid, and all drug is flushed by the flow to provide complete drug delivery.

The drug release or microchip release unit, whether configured in a static release configuration or a powered unit subject to active control by a microcontroller or other circuit, may be readily configured to administer multiple drugs in a drug cocktail, with the times and concentrations of each element accurately controlled. The delivery at higher than normal pressure at the distal catheter assures an increased rate of drug penetration of the target site, for example, within the parenchyma of the brain, while the use of a physiologically inert carrier as the pump refill enhances overall safety. By configuring the drug reservoirs in the wall of the catheter (or further by providing the reservoir portion as a separate short replaceable length or segment of the catheter) a defined drug cocktail may be provided with the time and durations of administration of its components accurately fixed, and their delivery to the exact target site assured. Advantageously, extended treatment regimens may thus be implemented, requiring a return office visit only to replenish the pump's carrier fluid reservoir (for example, when using a transdermally-refilled bellows-type infusion pump embodiment).

Figure 2A illustrates another construction similar to that of Figure 2. In this embodiment, a carrier pump 120' connects to a delivery catheter 140', and a release cartridge 130' fits within the catheter to provide a controlled release of one or more drugs or bioactive materials into the pumped carrier fluid. The release cartridge 130' may be solid body made of a drug with a solid binder that releases the drug at a defined rate, or may be a construct such as a permeable-walled cylinder that is loaded with liquid or solid treatment material and controls release of the material via the permeability of its walls. The replaceable release cartridge may have a single active agent or may contain a "cocktail" of agents, and may be compounded with different binders, particle coatings and the like to regulate the release of different agents at different concentrations, rates and/or times. In this regard, the cartridge 130' may be situated within a portion of the catheter body catheter 140' having a defined dimension and fluid capacity, and the cartridge may be formulated such that it quickly reaches equilibrium solvation, or otherwise attains a defined endpoint or concentration in the surrounding (known) volume of carrier fluid. This allows the drug doses to be well controlled, and permits the remaining cartridge capacity to be reliably calculated as a simple function of pumping cycles, enhancing the precision of long-term drug administration and treatment. In particular, such precise control of the cartridge lifetime assures that the cartridge will not be unnecessarily replaced before it is exhausted (which would result in excessive minor surgeries) nor will it remain implanted after being prematurely exhausted (which would result in a period of lack of treatment drug).

It is not necessary that the controlled release drug unit be situated directly in the delivery line as shown in the foregoing Figures; rather, it may communicate with the delivery line. Figure 1B illustrates an system 10' wherein the release unit 30' is in fluid communication with the delivery line 40' from the infusion pump 20 at a line junction 41. In this embodiment, the release unit may be fabricated with a source or mechanism for providing a small but sufficient flow of fluid to move the released drug along a junction line 42 into the delivery tube 40'. For example, it may have a permeable membrane on a side away from the junction line 42 to provide osmotic ingress of fluid, or other suitable means for moving it's released drug along the junction line 42.

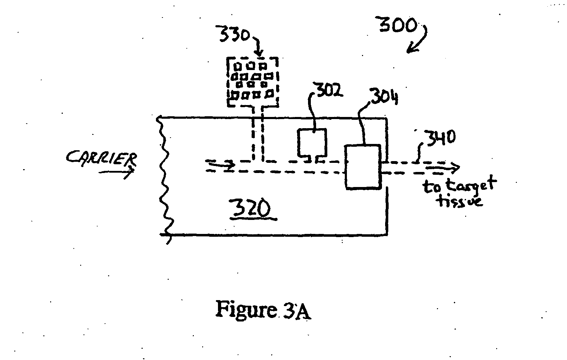

In accordance with another embodiment of the invention, systems may be configured to utilize a native bodily fluid as the pump's carrier fluid. The native bodily fluid may, for example, be the body's cerebrospinal fluid (CSF) when the targeted tissue is in the central nervous system. In this embodiment, the infusion pump need not necessarily possess a reservoir. Figure 3 schematically illustrates such a system 200.

As shown in Figure 3, a system 200 in accordance with this embodiment of the invention includes a pump 220 that has an inlet port connected to an inlet catheter uptake assembly 215 that resides in the patient's cerebrospinal fluid. The inlet catheter may, for example, be positioned near the base of the skull in the occipital region, and may have a suitable sump or head structure into which the CSF infiltrates. The CSF passes along the inlet catheter into the pump, and is actively pumped at the selected rate and times to the drug release unit 230 and along the delivery catheter 240 to the target tissue site. The release unit and delivery catheter may be separate, or the release unit may be integrated in the catheter or into the catheter wall as described above in regard to Figures 2 and 2A. The inlet catheter may be positioned in the sub-arachnoid space of the brain or spine, or other suitable position in the CNS. Preferably the inlet catheter assembly 215 draws fluid from a region sufficiently remote from the target tissue site, and at a rate such that the withdrawal of CSF does not create a negative or counter-acting pressure gradient that would alter or have any adverse effect on the convective drug delivery to the target tissue site at the outlet of the delivery catheter 240. That is, the inlet is positioned so that it does not counteract the positive gradient produced by the outlet(s) of the delivery catheter(s), or steal drug from the convectively-enhanced transport into parenchymal or other CNS tissue that occurs at the target site.

Systems of the invention may also be implemented to use other endogenous fluids as the carrier, such as blood, blood serum or lymphatic fluids, in which case the inlet catheter is positioned accordingly to collect such other fluid.

Figure 3A schematically illustrates other features of a further system 300 of the present invention. As shown, system 300 has a pump assembly 320 that pumps fluid (either from an inlet as described above or from a reservoir) into a delivery catheter 340. The pump assembly includes or communicates with a chamber 302 that contains a concentrated delivery agent. Chamber 302 supplies the agent at an appropriate dilution in the carrier fluid. The delivery agent may, for example, be a morphine, and the pump may deliver its output to the intrathecal space for pain management. Alternatively, the delivery agent may be another drug, adjuvant or the like.

System 300 also has a mixing chamber 304 in line with the fluid pumping path. Fluid carrying drug released by the chamber 302 and/or released by the controlled release device 330 mixes in the chamber 304 before entering the delivery catheter 340. This is a particularly advantageous construction for delivering a drug that must have a defined concentration, or for delivering pairs of drugs that must be combined shortly before delivery. The mixing chamber may be connected to receive material directly from the sources 302, 330, or it may be shaped and dimensioned to passively mix (e.g., by turbulence, or by solution) the fluid in the fluid path. In some embodiments, the mixing chamber may be provided with one or more additional openings and interconnections between ports, and these are coordinated with the pump or the flow in the delivery line to recirculate fluid through the chamber and enhance active mixing.

Thus, systems of the invention advantageously provide a drug or multi-drug infusion system that achieves enhanced delivery while employing a simple pump that advantageously uses a single, inert carrier fluid, and coordinates or couples its delivery with a controlled release drug device. By separating the physical delivery parameters via a pump mechanism from the substance/dose aspects of medication via the controlled release unit, the system provides a robust system that achieves enhanced drug distribution in the target tissue. It also permits various modular forms that result in more accurate implementation of multi-drug and/or multi-rate treatment regimens, as well as regimens having a varying range or schedule responsive to sensor measurements. It also provides a system architecture in which upstream bulk flow components may be more accessible and reduce the potential for indwelling incidents of sepsis or adverse physiological reaction. Moreover, while some drugs may suitably be compounded in the carrier fluid itself, the controlled drug release device enables the use of a wide range of other drugs which, by way of example, may be too unstable for long term storage in solution. Thus, the architectures of the present invention in those cases effectively compound the drug for delivery at the time of release, potentially augmenting the pharmacopoeia available for implanted delivery systems.

When the controlled released unit is a powered unit that relies on applying electrical signals to initiate release from each reservoir at appropriate times, these release signals may be coordinated with the pumping intervals to maximize the drug concentration in pumped fluid for the selected volume and rate of convective delivery. In particular, by initiating drug release into the restricted space of the flow path before starting pumping of the carrier, a highly concentrated fluid is delivered at a precise time and at an overpressure condition at the catheter outlet, without slow ramping-up of the release characteristics, and without allowing diminution by the body's initial clearance or breakdown mechanisms such as occurs in the prior art release devices. Thus, coordinating the release and the pumping cycles may maximize the initial concentration as well as the rate of transport into the target tissue site. Moreover, when utilizing an electrically controlled infusion pump, the timing and control signals for the release unit may be provided from the pump controller, allowing a range of modular constructions wherein a release unit having particular drugs or treatment materials "plugs in" to a pump unit having the desired delivery characteristics, and also having suitable control cycles programmed therein.

It will be understood that the term "drug" as used herein and in the attached claims refers not simply to complex organic chemicals, but is intended to mean any biologically relevant material that is to be delivered to a target tissue site. As such, it may include pharmaceutical compounds; treatment organisms; treatment fluids; cellular products, components or materials; label or probe material; and genetic sequence material among others. Furthermore, the term "carrier fluid" may be any compatible fluid that may be pumped at a rate effective to provide convective delivery of the drug into tissue. As such, it may be a fluid such as a physiological buffer or saline solution, an endogenous fluid or component thereof, such as blood plasma or cerebrospinal fluid. It may also involve various pharmaceutical preparations, such as excipients and adjuvants, or a combination of different ones of the foregoing fluids.

## Claims

1. An implantable drug delivery system (10) comprising:
an infusion pump (20) including a fluid outlet;
a first catheter (40) extending directly from the fluid outlet to a discharge portion positionable directly at a target tissue site; and
a controlled release drug assembly (30) configured for controllably releasing drug material,
wherein:
said drug assembly (30) is in direct communication with said first catheter (40) such that the drug material is released directly into said first catheter (40);
the pump (20) is effective to deliver a carrier fluid to the fluid outlet such that the drug material released into the first catheter (40) discharges directly at the discharge portion to treat the target tissue site;
the drug assembly (30) is in fluid communication with the first catheter (40) intermediate the pump and the target tissue site; and
the system being **characterised in that** it includes a controller (32a) configured to initiate drug release out of phase with the operation of the infusion pump (20) such that the drug material is released into the first catheter (40) before flow in the first catheter (40) is initiated.

2. The system of claim 1, wherein the pump further comprises a power source.

3. The system of claim 1 or claim 2, wherein the pump includes a chamber (24) for holding a predetermined quantity of carrier fluid.

4. The system of any one of claims 1 to 3, wherein the pump further includes an inlet line for delivering said carrier fluid to the pump, said pump being effective to convey the fluid from the inlet to the outlet.

5. The system of claim 4, wherein the inlet line includes a second, separate catheter (215) positionable in tissue for delivering an endogenous fluid to the pump.

6. The system of claim 5, wherein the second, separate catheter, the pump and the first catheter are dimensioned for positioning in tissue to form an endogenous fluid circulation loop.

7. The system of claim 5 or claim 6, wherein the second, separate catheter is configured to collect endogenous fluid from a donor site in the central nervous system, the circulatory system or the lymphatic system.

8. The system of any one of claims 1 to 7, wherein the drug assembly (30) is located in the first catheter.

9. The system of any one of claims 1 to 7, wherein the drug assembly (30') is located outside the first catheter.

10. The system of any one of claims 1 to 9, wherein the carrier fluid is a physiological buffer, a pharmaceutical excipient or adjuvant, an endogenous fluid or a combination thereof.

11. The system of claim 10, wherein the carrier fluid is an endogenous fluid which is cerebral spinal fluid, blood, lymphatic fluid, a component thereof or a combination thereof.

12. The system of any one of claims 1 to 11, wherein the infusion pump (20) is effective to deliver carrier fluid at a rate effective to induce convective bulk transport of the drug into tissue at the target site.

13. The system of claim 12, wherein the flow rate ranges from 0.5 to 20 microliters per minute.

14. The system of any one of claims 1 to 13, wherein the infusion pump (20) is operable to continuously maintain enhanced fluid pressure over a predetermined period of time.

15. The system of any one of claims 1 to 14, wherein the infusion pump (20) is a pressurized reservoir, a peristaltic pump, a diaphragm pump or a piston pump.

16. The system of any one of claims 1 to 15, wherein the infusion pump (20) includes a microcontrol unit that controls flow rate of the pump.

17. The system of claim 16, wherein the microcontrol unit controls fluid delivery pressure profile over a predetermined period of time.

18. The system of any one of claims 1 to 17, wherein the drug assembly (30) is a microchip having at least one drug reservoir (31, 33, 35).

19. The system of claim 18, wherein the microchip contains one or more drugs therein.

20. The system of claim 19, wherein the one or more drugs are released in a pulsatile, intermittent or continuous delivery regimen.

21. The system of claim 18, wherein there is a plurality of reservoirs (31, 33, 35) containing plural different drugs, drug concentrations or a combination thereof.

22. The system of any one of claims 18 to 21, wherein the microchip is powered by a power source.

23. The system of any one of claims 18 to 22, when dependent on claim 17 or claim 18, wherein the microchip is in communication with the microcontrol unit.

24. The system of claim 23, wherein the microchip is in communication with the microcontrol unit through a microprocessor (32a).

25. The system of any one of claims 18 to 24, wherein the reservoir (31, 33, 35) has a cap positioned over a drug contained therein, wherein release of the drug is controlled by diffusion through or disintegration of the cap.

26. The system of claim 25 when dependent on claim 24, wherein diffusion through or disintegration of the cap is controlled by the microprocessor (32a).

27. The system of any one of claims 1 to 26, further comprising one or more biosensors (S) wherein the system responds to a biosensor signal.

28. The system of claim 27, wherein the biosensor (S) is in at least one of the first catheter (40), the tissue site and the drug assembly (30), for controlling the infusion pump (20) and/or the drug assembly (30) in response to biosensor signals.

29. The system of any one of claims 1 to 28, further comprising an array of biosensors (S) for disposition in tissue wherein the infusion pump and/or the drug assembly responds to biosensor signals from the array.

30. The system of claim 29, further including means for detecting a material or condition with a biosensor array, and for controlling the infusion pump (20) and/or the drug assembly (30) in response thereto.

31. The system of any one of claims 1 to 30, wherein the drug assembly (30) includes plural controllable release sites (130a, 130b, 130c, 130d) positioned within a wall of the first catheter (40).

32. The system of any one of claims 1 to 31, wherein the target site is brain tissue.

33. The system of any one of claims 1 to 32, wherein the first catheter (40) terminates in a distal end, wherein the distal end is implantable within the target-site.

## Patentansprüche

1. Implantierbares System (10) zur Arnzeimittelverabreichung, welche Folgendes umfasst:
eine Infusionspumpe (20), welche einen Fluidauslass aufweist;
einen ersten Katheter (40), welcher sich direkt von dem Fluidauslass zu einem Austrittssteilbereich erstreckt, welcher direkt an einem Zielgewebsort positionierbar ist; und
eine regulierte Arzneimittelfreigabeanordnung (30), welche eingerichtet ist, um regulierbar Arzneimittelmaterial freizugeben,
wobei:
die Arzneimittelanordnung (30) in direkter Verbindung mit dem ersten Katheter (40) ist, so dass das Arzneimittelmaterial direkt in den ersten Katheter (40) freigegeben wird;
die Pumpe (20) wirksam ist, um dem Fluidauslass ein Trägerfluid zuzuführen, so dass das Arzneimittelmaterial, welches in den ersten Katheter (40) freigegeben wird, direkt an dem Austrittsteilbereich austritt, um den Zielgewebsort zu behandeln;
die Arnzeimittelanordnung (30) in Fluidverbindung mit dem ersten Katheter (40) zwischen der Pumpe und dem Zielgewebsort ist; und wobei das System **dadurch gekennzeichnet ist, dass** es eine Steuerungseinheit (32a) aufweist, welche eingerichtet ist, eine Arzneimittelfreigabe phasenverschoben zu dem Betrieb der Infusionspumpe (20) auszulösen, so dass das Arzneimittelmaterial in den ersten Katheter (40) freigegeben wird, bevor ein Fluss in dem ersten Katheter (40) ausgelöst wird.

2. System nach Anspruch 1, wobei die Pumpe weiterhin eine Energiequelle umfasst.

3. System nach Anspruch 1 oder 2, wobei die Pumpe eine Kammer (24) zum Halten einer vorbestimmten Menge eines Trägerfluids aufweist.

4. System nach einem der Ansprüche 1 bis 3, wobei die Pumpe weiterhin eine Einlassleitung aufweist, um der Pumpe das Trägerfluid zuzuführen, wobei die Pumpe wirksam ist, um das Fluid von dem Einlass zu dem Auslass zu befördern.

5. System nach Anspruch 4, wobei die Einlassleitung einen zweiten, gesonderten Katheter (215) aufweist, welcher in Gewebe positionierbar ist, um der Pumpe ein endogenes Fluid zuzuführen.

6. System nach Anspruch 5, wobei der zweite, gesonderte Katheter, die Pumpe und der erste Katheter für einen Positionieren in Gewebe bemessen sind, um eine Zirkulationsschleife für ein endogenes Fluid zu bilden.

7. System nach Anspruch 5 oder 6, wobei der zweite, gesonderte Katheter eingerichtet ist, um endogenes Fluid von einem Spenderort in dem zentralen Nervensystem, dem Kreislaufsystem oder dem lymphatischen System zu sammeln.

8. System nach einem der Ansprüche 1 bis 7, wobei sich die Arzneimittelanordnung (30) in dem ersten Katheter befindet.

9. System nach einem der Ansprüche 1 bis 7, wobei sich die Arzneimittelanordnung (30') außerhalb des ersten Katheters befindet.

10. System nach einem der Ansprüche 1 bis 9, wobei das Trägerfluid ein physiologischer Puffer, ein pharmazeutischer Hilfsstoff oder Zusatz, ein endogenes Fluid oder eine Kombination davon ist.

11. System nach Anspruch 10, wobei das Trägerfluid ein endogenes Fluid ist, welches zerebrales Spinalfluid, Blut, lymphatisches Fluid, ein Bestandteil von diesen oder eine Kombination von diesen ist.

12. System nach einem der Ansprüche 1 bis 11, wobei die Infusionspumpe (20) wirksam ist, um ein Trägerfluid mit einer Geschwindigkeit zuzuführen, welche wirksam ist, einen konvektiven Volumentransport des Arzneimittels in das Gewebe an dem Zielort herbeizuführen.

13. System nach Anspruch 12, wobei die Flussgeschwindigkeit im Bereich von 0,5 bis 20 Mikroliter pro Minute liegt.

14. System nach einem der Ansprüche 1 bis 13, wobei die Infusionspumpe (20) funktionsbereit ist, um fortlaufend einen erhöhten Fluiddruck über eine vorbestimmte Zeitperiode aufrechtzuerhalten.

15. System nach einem der Ansprüche 1 bis 14, wobei die Infusionspumpe (20) ein Behälter unter Druck, eine peristaltische Pumpe, eine Membranpumpe oder eine Kolbenpumpe ist.

16. System nach einem der Ansprüche 1 bis 15, wobei die Infusionspumpe (20) eine Mikrosteuerungseinheit aufweist, welche eine Flussgeschwindigkeit der Pumpe reguliert.

17. System nach Anspruch 16, wobei die Mikrossteuerungseinheit ein Fluidzuführungsdruckprofil über eine vorbestimmte Zeitdauer reguliert.

18. System nach einem der Ansprüche 1 bis 17, wobei die Arzneimittelanordnung (30) ein Mikrochip ist, welcher zumindest einen Arzneimittelbehälter (31, 33, 35) aufweist.

19. System nach Anspruch 18, wobei der Mikrochip ein oder mehrere Arzneimittel in sich beinhaltet.

20. System nach Anspruch 19, wobei das eine oder mehrere Arzneimittel in einem pulsierenden, intermittierenden oder kontinuierlichen Zuführungsbetriebszustand freigegeben werden.

21. System nach Anspruch 18, wobei eine Vielzahl von Behältern (31, 33, 35) vorhanden ist, welche eine Vielzahl unterschiedlicher Arzneimittel, Arzneimittelkonzentrationen oder Kombinationen davon beinhalten.

22. System nach einem der Ansprüche 18 bis 21, wobei der Mikrochip von einer Energiequelle mit Energie versorgt wird.

23. System nach einem der Ansprüche 18 bis 22, wenn dieser von Anspruch 17 oder Anspruch 18 abhängig ist, wobei sich der Mikrochip in Kommunikation mit der Mikrosteuerungseinheit befindet.

24. System nach Anspruch 23, wobei sich der Mikrochip mit der Mikrosteuerungseinheit durch einen Mikroprozessor (32a) in Kommunikation befindet.

25. System nach einem der Ansprüche 18 bis 24, wobei der Behälter (31, 33, 35) einen Deckel aufweist, welcher über einem Arzneimittel, welches darin beinhaltet ist, positioniert ist, wobei eine Freigabe des Arzneimittels durch eine Diffusion durch den Deckel oder dessen Desintegration reguliert ist.

26. System nach Anspruch 25, wenn dieser von Anspruch 24 abhängig ist, wobei die Diffusion durch den Deckel oder dessen Desintegration durch den Mikroprozessor (32a) reguliert ist.

27. System nach einem der Ansprüche 1 bis 26, welches weiterhin einen oder mehrere Biosensoren (S) umfasst, wobei das System auf ein Biosensorsignal reagiert.

28. System nach Anspruch 27, wobei der Biosensor (S) sich zumindest in dem ersten Katheter (40), dem Gewebsort und/oder der Arzneimittelanordnung (30) befindet, um die Infusionspumpe (20) und/oder die Arzneimittelanordnung (30) in Reaktion auf Biosensorsignale zu regulieren.

29. System nach einem der Ansprüche 1 bis 28, welches weiterhin eine Gruppe von Biosensoren (S) für eine Anlage in Gewebe umfasst, wobei die Infusionspumpe und/oder die Arzneimittelanordnung auf Biosensorsignale der Gruppe reagiert.

30. System nach Anspruch 29, welches weiterhin ein Mittel zum Erkennen eines Materials oder einer Bedingung mit einer Biosensorgruppe und zum Regulieren der Infusionspumpe (20) und/oder der Arzneimittelanordnung (30) in Reaktion hierauf aufweist.

31. System nach einem der Ansprüche 1 bis 30, wobei die Arzneimittelanordnung (30) eine Vielzahl regulierbarer Freigabeorte (130a, 130b, 130c, 130d) aufweist, welche innerhalb einer Wand des ersten Katheters (40) positioniert sind.

32. System nach einem der Ansprüche 1 bis 31, wobei der Zielort Himgewebe ist.

33. System nach einem der Ansprüche 1 bis 32, wobei der erste Katheter (40) mit einem distalen Ende abschließt, wobei das distale Ende innerhalb des Zielortes implantierbar ist.

## Revendications

1. Système implantable d'administration de médicament (10) comprenant :
• une pompe de perfusion (20) comprenant un orifice de sortie de fluide ;
• un premier cathéter (10) s'étendant directement de l'orifice de sortie de fluide à une partie de décharge positionnable directement au niveau d'un site de tissu cible ; et
• un ensemble de médicament à libération contrôlée (30) configuré pour libérer un médicament de façon contrôlable,
dans lequel :
• ledit ensemble de médicament (30) est en communication directe avec ledit premier cathéter (40) de telle sorte que le médicament soit libéré directement dans ledit premier cathéter (40) _{;}
• la pompe (20) permet de délivrer un fluide porteur à l'orifice de sortie de fluide de telle sorte que le médicament libéré dans le premier cathéter (40) se décharge directement au niveau de la partie de décharge pour traiter le site de tissu cible;
• l'ensemble de médicament (30) est en communication fluide avec le premier cathéter (10) entre la pompe et le site de tissu cible ; et
• le système étant **caractérisé en ce qu'**il comprend un contrôleur (32a) configure pour lancer la libération de médicament de façon décalée avec le fonctionnement de la pompe de perfusion (20) de telle sorte que le médicament soit libéré dans le premier cathéter (10) avant le début de l'écoulement dans le premier cathéter (40).

2. Système selon la revendication 1, dans lequel la pompe comprend en outre une source d'alimentation.

3. Système selon la revendication 1 ou la revendication 2, dans lequel la pompe comprend une chambre (24) destinée à contenir une quantité prédéterminée de fluide porteur.

4. Système selon l'une quelconque des revendications 1 à 3, dans lequel la pompe comprend en outre un conduit d'entrée pour délivrer ledit fluide porteur à la pompe, ladite pompe permettant d'acheminer le fluide de l'orifice d'entrée à l'orifice de sortie.

5. Système selon la revendication 4, dans lequel le conduit d'entrée comprend un second cathéter séparé (21b) positionnable dans le tissu destiné à délivrer un fluide endogène à la pompe.

6. Système selon la revendication 5, dans lequel le second cathéter séparé, la pompe et le premier cathéter sont dimensionnés pour être positionnée dans le tissu pour former une boucle de circulation de fluide endogène.

7. Système selon la revendication 5 ou la revendication 6, dans lequel le second cathéter séparé est configuré pour collecter du fluide endogène provenant d'un site donner dans le système nerveux central, le système circulatoire ou le système lymphatique.

8. Système selon l'une quelconque des revendications 1 à 7, dans lequel l'ensemble de médicament (30) se trouve dans le premier cathéter.

9. Système selon l'une quelconque des revendications 1 à 7, dans lequel l'ensemble de médicament. (30') se trouve à l'extérieur du premier cathéter.

10. Système selon l'une quelconque des revendications 1 à 9, dans lequel le fluide porteur est un tampon physiologique, un excipient ou adjuvant pharmaceutique, un fluide endogène ou une combinaison de ceux-ci.

11. Système selon la revendication 10, dans lequel le fluide porteur est un fluide endogène qui est un fluide céphalorachidien, du sang, un fluide lymphatique, une composante ou une combinaison de ceux-ci.

12. Système selon l'une quelconque des revendications 1 à 11, dans lequel la pompe de perfusion (20) permet de délivrer un fluide porteur à une vitesse efficace pour induire le transport de masse convectif du médicament dans le tissu au niveau du site cible.

13. Système selon la revendication 12, dans lequel le débit varie de 0,5 à 20 microlitres par minute.

14. Système selon l'une quelconque des revendications 1 à 13, dans lequel la pompe de perfusion (20) est utilisable pour maintenir en continu une pression de fluide améliorée sur une période de temps prédéterminée.

15. Système selon l'une quelconque des revendications 1 à 14, dans lequel la pompe de perfusion (20) est un réservoir pressurisé, une pompe péristaltique, une pompe à diaphragme ou une pompe à piston .

16. Système selon l'une quelconque des revendications 1 à 15, dans lequel la pompe de perfusion (20) comprend une unité de microcontrôle qui contrôle le débit de la pompe.

17. Système selon la revendication 16, dans lequel l'unité de microcontrôle contrôle le profil de pression d'administration de fluide sur une période de temps prédéterminée.

18. Système selon l'une quelconque des revendications 1 à 17, dans lequel l'ensemble de médicament (30) est une microplaquette ayant au moins un réservoir de médicaments.

19. Système selon la revendication 18, dans lequel la microplaquette contient un ou plusieurs médicaments.

20. Système selon la revendication 19, dans lequel le ou les médicaments sont libérés selon un régime d'administration pulsatile, intermittent ou continu.

21. Système selon la revendication 18, dans lequel il existe une pluralité de réservoirs (31, 33, 35) contenant plusieurs médicaments différents ou une combinaison de ceux-ci.

22. Système selon l'une quelconque des revendications 18 à 21, dans lequel la microplaquette est alimentée par une source d'alimentation.

23. Système selon l'une quelconque des revendications 18 à 22, lorsqu'elles sont subordonnées à la revendication 17 ou de la revendication 18, dans lequel la microplaquette est en communication avec l'unité de microcontrôle.

24. Système selon la revendication 23, dans lequel la microplaquette est en communication avec l'unité de microcontrôle par le biais d'un microprocesseur (32a).

25. Système selon l'une quelconque des revendications 18 à 24, dans lequel le réservoir (31, 33, 35) possède un bouchon positionné sur un médicament contenu dans le réservoir, dans lequel la libération du médicament est contrôlée par diffusion à travers le bouchon ou désintégration de celui-ci.

26. Système selon la revendication 25, lorsqu'elle est subordonnée à la revendication 24, dans lequel la diffusion à travers le bouchon ou la désintégration de celui-ci est contrôlée par le microprocesseur (32a).

27. Système selon l'une quelconque des revendications 1 à 26, comprenant en outre un ou plusieurs biocapteurs (S), dans lequel le système répond à un signal de biocapteur.

28. Système selon la revendication 27, dans lequel le biocapteur (S) se trouve dans au moins un du premier cathéter (40), du site tissulaire et de l'ensemble de médicament (30), pour contrôler la pompe de perfusion (20) et/ou l'ensemble de médicament (30) en réponse aux signaux de biocapteur.

29. Système selon l'une quelconque des revendications 1 à 28, comprenant en outre un réseau de biocapteurs (S) destinés à être disposés dans le tissu, dans lequel la pompe de perfusion et/ou l'ensemble de médicament répond(ent) aux signaux des biocapteurs du réseau.

30. Système selon la revendication 29, comprenant en outre des moyens pour détecter une matière ou un état avec un réseau de biocapteurs, et pour contrôler en réaction la pompe de perfusion (20) et/ou l'ensemble de médicament (30).

31. Système selon l'une quelconque des revendications 1 à 30, dans lequel l'ensemble de médicament (30) comprend plusieurs sites de libération contrôlables (130a, 130b, 130c, 130d) positionnés à l'intérieur d'une paroi du premier cathéter (40).

32. Système selon l'une quelconque des revendications 1 à 31, dans lequel le site cible est un tissu de cerveau.

33. Système selon l'une quelconque des revendications 1 à 32, dans lequel le premier cathéter (40) se termine à une extrémité distale, l'extrémité distale étant implantable à l'intérieur du site cible.
